# EUROPEAN PATENT SPECIFICATION

(11) **EP 3 554 524 B1**
(45) Date of publication and mention of the grant of the patent: **20.01.2021**
(21) Application number: 17817713.5
(22) Date of filing: 12.12.2017
(51) Int. Cl.: A61K 36/88, A61K 31/355, A61K 36/899, A61K 36/15, A23L 33/105, A61P 25/24

(54) **COMPOSITION FOR USE IN TREATING DYSPHORIA, DEPRESSION AND/OR MOOD SWINGS RELATING TO PREMENSTRUAL SYNDROME (PMS)**
ZUSAMMENSETZUNG ZUR VERWENDUNG BEI DER BEHANDLUNG VON SYMPTOMEN IM ZUSAMMENHANG MIT PRÄMENSTRUELLEM SYNDROM (PMS)
COMPOSITION DESTINÉE À ÊTRE UTILISÉE DANS LE TRAITEMENT DES SYMPTÔMES RELATIFS AU SYNDROME PRÉMENSTRUEL (PMS)

(30) Priority: 13.12.2016 EP 16203846
(43) Date of publication of application: 23.10.2019
(73) Proprietor: Serelys Pharma S.A.M., 98000 Monaco (MC)
(72) Inventor: MORRA, Sossio, 98000 Monaco (MC)
(74) Representative: Axe PI
(86) International application number: PCT/EP2017/082371
(87) International publication number: WO 2018/108879

(56) References cited:
- WO-A1-02/17944
- CN-A- 104 293 542
- CN-B- 102 028 842
- FR-A1- 2 982 161
- "FEMAL NATURAL HELP FOR PMS", INTERNET CITATION, 1999, XP002906801, Retrieved from the Internet: URL:HTTP://WWW.SISUHEALTH.COM/PRODUCTS/FEM AL.HTML [retrieved on 2001-04-19]
- DATABASE WPI Week 201676 Thomson Scientific, London, GB; AN 2016-600198 XP002769577, & CN 105 918 864 A (HEFEI ZHUGUANG GRAIN & OIL TRADE CO LTD) 7 September 2016 (2016-09-07)
- DATABASE WPI Week 201430 Thomson Scientific, London, GB; AN 2014-E84922 XP002769578, & CN 103 504 371 A (YANG R) 15 January 2014 (2014-01-15)
- K WINTHER ET AL: "Femal, a herbal remedy made from pollen extracts, reduces hot flushes and improves quality of life in menopausal women: a randomized, placebo-controlled, parallel study", CLIMACTERIC, vol. 8, no. 2, 3 June 2005 (2005-06-03), pages 162-170, XP055354258, GB ISSN: 1369-7137, DOI: 10.1080/13697130500117987
- DATABASE WPI Week 201657 Thomson Scientific, London, GB; AN 2016-42991F XP002769640, & ES 2 573 542 A1 (PHARMACTIVE BIOTECH PROD SL) 8 June 2016 (2016-06-08)
- AGHA-HOSSEINI M ET AL: "Crocus sativus L. (saffron) in the treatment of premenstrual syndrome: A double-blind, randomised and placebo-controlled trial", BJOG: AN INTERNATIONAL JOURNAL OF OBSTETRICS AND GYNAECOLOGY 200803 GB, vol. 115, no. 4, March 2008 (2008-03), pages 515-519, XP002769579, ISSN: 1470-0328 cited in the application
- PIRDADEH BEIRANVAND SOHEILA ET AL: "The effect ofCrocus sativus(saffron) on the severity of premenstrual syndrome", EUROPEAN JOURNAL OF INTEGRATIVE MEDICINE, ELSEVIER, AMSTERDAM, NL, vol. 8, no. 1, 2 July 2015 (2015-07-02), pages 55-61, XP029469376, ISSN: 1876-3820, DOI: 10.1016/J.EUJIM.2015.06.003
- TERMAN A ET AL: "The effect of polbax extract on lipofuscin accumulation in cultured neonatal rat cardiac myocytes", MEDICINAL & AROMATIC PLANTS ABSTRACTS, SCIENTIFIC PUBLISHERS, SCIENTIFIC PUBLISHERS, NEW DELHI - INDIA, vol. 24, no. 6, 1 December 2002 (2002-12-01), XP018011987, ISSN: 0250-4367

## Description

### TECHNICAL FIELD

The invention pertains to the technical field of compositions for use in treating symptoms relating to PMS.

### BACKGROUND

The term premenstrual syndrome, or PMS, is generally used to describe a group of physical and mental symptoms which occur cyclically beginning about seven to fourteen days prior to menses. Menstruation occurs in women from the age of about twelve to thirteen to, on average, until about forty-seven years of age. It occurs at more or less regular intervals except during pregnancy and lactation. The normal menstrual cycle averages about twenty-eight days with some variation based upon the woman's genetic makeup, age, physical and emotional well-being, as well as other factors. The duration of menstrual flow is variable but usually is between three and seven days. The symptoms of PMS are often so severe and widespread that the American Psychiatric Association has formally identified the diagnostic criteria for PMS in Diagnostic and Statistical Manual of Mental Disorders.

The specific etiology of PMS remains unknown, although many theories have been proposed. These theories include, but are not limited to: hormonal imbalances, hormonal deficiencies, vitamin deficiencies, disturbances of autonomic nervous system, salt and water imbalances, altered endogenous opiates such as endorphins and psychosomatic dysfunction, just to name a few. However, up to now, investigative studies of etiology have been inconclusive and sometimes conflicting. Most likely, PMS is multifactorial and probably also involves changes in neurohormones and neurotransmitters, which are difficult to observe, document and isolate in vivo.

Symptoms of PMS are varied and can range from mild to incapacitating. As many as seventy to ninety percent of all women have recurrent premenstrual syndrome, and as many as twenty to forty percent of these women suffer some degree of temporary physical and/or mental incapacitation. Some examples of mental symptoms a woman suffering from PMS may exhibit include difficulty in concentration, fatigue, change in appetite, irritability and depression. Some examples of physical symptoms a woman suffering from PMS may exhibit are increase or decrease in sleep, joint pain, cramps, bloating, edema, acne, constipation and breast tenderness.

Compositions comprising extracts of pollen and/or pistils for combatting symptoms relating to PMS have been previously described in the art. As these compositions are all natural, they provide an attractive treatment for women suffering from PMS.

However, recent hitherto unknown studies have shown that although these compositions are indeed helpful in treating or alleviating a large part of symptoms relating to PMS, these compositions show less or no efficacy towards symptoms relating to depression and dysphoria. In fact, it was surprisingly reported in this novel study that in women for whom the most severe PMS symptom was depression and/or dysphoria, these pollen extract based compositions have almost no effect.

Hence, there is a need in the art for compositions which are able to combat all symptoms of PMS, including those that concern mood swings, forms of depression and general dysphoria. By preference, such composition is of natural origin.

In view of the above, a problem to be solved by the object of the present invention is to provide a composition which is suitable for combatting a broad range of a symptoms relating to PMS, including those directed to depression and dysphoria. The composition is an all-natural composition, and can be taken without any risk for unwanted side-effects.

### SUMMARY OF THE INVENTION

Consequently, a first object of the solution proposed by the invention to remedy this problem is to provide a composition for use in treating dysphoria, depression and/or mood swings relating to premenstrual syndrome (PMS) wherein said composition comprises as first active ingredient: - a pollen and pistil extract derived from plants belonging to the *Graminae* and/or *Pinacea* family, preferably from plant material originating from *Zea mays L.*, *Secale cereale L.*, *Dactylis glomerata L., Pinus sylvestris L*. or a mixture thereot, at least one second active ingredient, said second active ingredient being a source of satranal and/or (picro)crocin wherein said source of safranal and/ or (picro)crocin is saffron or a derivative of saffron, and- vitamin E. Such a composition provides an optimal, plant based treatment for treating and/or preventing a full range of symptoms relating to PMS, including dysphoria and depression. The composition is hence suitable for all patients reporting one or more PMS symptoms like dysphoria, depression and/or mood swings, and is not restricted to those reporting classical symptoms. As a consequence, the composition according to the present invention is broadly applicable and highly efficient.

### FIGURES

- Figure 1 (including Figures 1A-1C) shows the differences of effect observed in a patient group suffering from classic PMS symptoms (Figure 1B) and those suffering from depression and/or dysphoria (Figure 1C) when treated with a composition based on a pollen and pistil extract; and
- Figure 2 shows the differences in weight gain prior to the menses in a patient group suffering from classic PMS symptoms and those suffering from depression and/or dysphoria when treated with a composition based on a pollen and pistil extract.

### DETAILED DESCRIPTION OF THE INVENTION

The present invention concerns a novel composition for use in treating symptoms relating to premenstrual syndrome (PMS). The composition provides an effective solution for treating dysphoria, depression and/or mood swings relating to premenstrual syndrome (PMS) relating to PMS.

Unless otherwise defined, all terms used in disclosing the invention, including technical and scientific terms, have the meaning as commonly understood by one of ordinary skill in the art to which this invention belongs. By means of further guidance, term definitions are included to better appreciate the teaching of the present invention.

As used herein, the following terms have the following meanings:
"A", "an", and "the" as used herein refers to both singular and plural referents unless the context clearly dictates otherwise. By way of example, "a compartment" refers to one or more than one compartment.

"About" as used herein referring to a measurable value such as a parameter, an amount, a temporal duration, and the like, is meant to encompass variations of +/-20% or less, preferably +/-10% or less, more preferably +/-5% or less, even more preferably +/-1% or less, and still more preferably +/-0.1% or less of and from the specified value, in so far such variations are appropriate to perform in the disclosed invention. However, it is to be understood that the value to which the modifier "about" refers is itself also specifically disclosed.

"Comprise," "comprising," and "comprises" and "comprised of" as used herein are synonymous with "include", "including", "includes" or "contain", "containing", "contains" and are inclusive or open-ended terms that specifies the presence of what follows e.g. component and do not exclude or preclude the presence of additional, non-recited components, features, element, members, steps, known in the art or disclosed therein.

The recitation of numerical ranges by endpoints includes all numbers and fractions subsumed within that range, as well as the recited endpoints.

The expression "% by weight" (weight percent), here and throughout the description unless otherwise defined, refers to the relative weight of the respective component based on the overall weight of the composition.

The term "extract" is to be understood as the liquid substance resulting of an extraction process of raw material such as plant material, preferably by use of a solvent. For the purpose of the invention, the term "extract" equally applies to a component which is directly obtained from an extract, such as a spray dried extract.

The term "component of a composition" is to be understood as a constituent of the composition which is comprised of one or more active ingredients.

The term "excipient" is to be understood as a natural or synthetic substance formulated alongside the active ingredient(s) of composition included for the purpose of bulking-up compositions that contain active ingredients or to confer a therapeutic enhancement on the active ingredient in the final dosage form, such as facilitating drug absorption or solubility, or to aid in the manufacturing of the composition.

The term "flowing agent" is to be understood as a substance which is added to a (solid) formulation to improve its flowability.

The term "anticaking agent" is to be understood as a substance which prevents the formation of lumps (caking).

The term "binding agent" is to be understood as a substance which holds ingredients together, ensuring that tablets and granules can be formed.

The term "bulking agent" is to be understood as a substance which is a diluting agent or for providing substance to a formulation. A bulking agent is generally inactive.

The term "herbal" is to be understood as a component which is derived of or consists or comprises herbs as main constituent. Herbal derivatives are to be understood as components which are mainly derived from herbs or herbal constituents, such as but not limiting to extracts, powders, dried herbal parts or tinctures.

The term "botanical (material) derived component" is to be understood as a component which is mainly derived from a plant or plant parts, said derivatives may be extracts, powders, dried plant parts or tinctures.

In a first aspect, the present invention relates to a composition for use in treating dysphoria, depression and/or mood swings relating to premenstrual syndrome (PMS) wherein said composition comprises as first active ingredient: - a pollen and pistil extract derived from plants belonging to the *Graminae* and/or *Pinacea* family, preferably from plant material originating from *Zea mays L.*, *Secale cereale L.*, *Dactylis glomerata L.*, *Pinus sylvestris L*. or a mixture thereof, at least one second active ingredient, said second active ingredient being a source of safranal and/or (picro)crocin wherein said source of safranal and/or (picro)crocin is saffron or a derivative of saffron, and- vitamin E. Said second active ingredients is a herbal or botanical derived component suitable for treating depressive and/or dysphoria symptoms linked to PMS.

The inventors of the present invention have surprisingly found that compositions comprising an extract of pollen and optionally pistils, generally known in the art to be effective for alleviating or treating symptoms relating to PMS, are less efficient or in some patients even not useful when those PMS symptoms relate to depression, dysphoria and mood swings.

As a result, a subpopulation of patients seeking for a treatment or alleviation of their PMS related symptoms will not or only inefficiently be helped when treated with compositions comprising pollen extract alone. This was hitherto not known. Although the reason behind the lack of activity of the pollen extract formulations is not known, it is deemed that the reason can be found in a biochemical difference between the patient groups that report dysphoria, depression and/or mood swings as one of the symptoms of PMS. Women with dysphoria and depression as main concern do not retain water prior to menses and do not suffer from weight gain, whereas women who suffer from a more classical symptomatic pattern did report water retention. Hence, it is deemed that a biological and/or biochemical cause lies at the basis of the observed differences in patient groups and effectiveness in treatment of the pollen extract based formulations.

As a consequence, the inventors of the present invention sought and found a method of provide to PMS patients a composition that provides a solution for all PMS related symptoms, including those mainly observing a problem with dysphoria, depression and/or mood swings.

Said second active ingredient is a source of safranal and/or (picro)crocin, suitable for improving treatment of depressive and/or dysphoria symptoms linked to PMS.

However, by combination with the first active ingredient, a composition is provided which is able to treat or alleviate the majority if not all PMS related symptoms which are recognized today, including at least dysphoria and/or depression.

Safranal and (picro)crocin which are generally found in saffron, have been previously reported as being useful against treating or preventing depression and/or dysphoria, or symptoms relating to the latter disorders. For example, the usefulness of saffron or a saffron extract in treating or preventing symptoms relating to depression or dysphoria in PMS women has been described (see Agha-Hosseini et al., 2008, In international journal of obstetrics and gynaecology).

Nevertheless, the inventors of the present invention have found that by supplementing a composition comprising a pollen and pistil extract derived from plants belonging to the *Graminae* and/or *Pinacea* family with a (botanical) source of safranal and/or (picro)crocin, the majority of women, if not all, suffering from PMS symptoms, particularly those with depression, dysphoria and/or mood swings as main concern, can be treated or helped in an improved way.

Said second active ingredient is saffron or a derivative of saffron. Said derivative of saffron is more preferably a saffron extract, even more preferably derived from *Crocus sativus.* In a most preferred embodiment, said saffron extract is an extract of dried petal of *Crocus sativus.*

By preference, the amount of safranal and/or (picro)crocin in the saffron extract will be at least 0.1%; in a further, more preferred embodiment, said amount will be between 0.1 and 10%.

In another embodiment, the amount of safranal and/or (picro)crocin in the final composition will be at least 0.001% by weight of the total composition, more preferably between 0.001% and 1%. This amount was found sufficient to provide an optimal effect.

The composition of the invention further comprises a pollen and pistil extract, which is also known to act effectively against the vast majority of PMS related symptoms, except for depression and/or dysphoria. Said extract of pollen and pistil is derived from plants belonging to the *Graminae* and/or *Pinacea* family.

Preferably, these extracts originate from plant material originating from corn (*Zea mays L*.), rye (*Secale cereale L*.), *Dactylis glomerata L., Pinus sylvestris* L. or a mixture thereof.

Preferably, the plant material used for the extracts is freshly harvested. The pollens used for the invention can be pollen collected by insects (such as bee pollen) or collected by human intervention. Bee pollen for instance contains pollen, but also nectar and saliva from bees. Pollen collected via human intervention is devoid of such additional ingredients. By preference, said pollens of the compositions of the invention are obtained by human intervention only.

Advantageously, the composition of the invention comprises a component which is derived from a mixture of at least separate two extracts, whereby a first extract originates from corn (*Zea mays L*.) pistil and from pollen selected from corn (*Zea mays L*.), rye (*Secale cereale L.*)*,* cocksfoot (*Dactylis glomerata L*.), pine (*Pinus silvestris L*.) or any combination thereof; and a second extract originating from corn (*Zea mays L*.) pollen and corn (*Zea mays L*.) pistil.

Preferably, said first extract originates from a mixture of at least corn (*Zea mays L*.) pollen, rye (*Secale cereale L.*) pollen, cocksfoot (*Dactylis glomerata L*.) pollen, pine (*Pinus silvestris L*.) pollen and corn (*Zea mays L*.) pistil.

In another embodiment, said composition comprises a first and a second extracts, whereby said first extract is originating from pollen selected from corn (*Zea mays L*.), rye (*Secale cereale L.*)*,* pine (*Pinus silvestris L*.) or a combination thereof and a second extract, whereby said second extract is originating from corn (*Zea mays L*.) pistil and pollen selected from corn (*Zea mays L*.), rye (*Secale cereale L.*)*,* cocksfoot (*Dactylis glomerata L*.), pine (*Pinus silvestris L*.) or any combination thereof.

The inventors found that the best results were obtained by making use of pollen and pistil extract derived from a mixture of plants.

By preference, the concentration of first active ingredient, being the pollen and pistil extract, in the composition is the same or higher than the concentration of the second active ingredient, which is a source of safranal and/or (picro)crocin suitable for treating depressive and/or dysphoria symptoms linked to PMS.

By preference, said first and second ingredients will be present at a specific ratio. Preferably, the ratio between said first and second active ingredients in said composition is between 1:1 and 20:1, more preferably between 1:1 and 10:1.

The active ingredients of plant origin are preferably comprised at least at 35% by weight of the total composition, more preferably at least at 40% by weight of the total composition, more preferably at between 40 and 75% by weight of the total composition. The latter ensures optimal activity of the active ingredients in the composition.

In a preferred embodiment, said composition is free of phytoestrogens and/or does not exhibit any phytoestrogenic activity. Phytoestrogens are naturally-occurring plant compounds that are structurally and/or functionally similar to mammalian estrogens and their active metabolites. Due to their similarities with estrogens, phytoestrogens have been reported to act as antagonists of estrogens. Simultaneously to this antagonistic effect, they are also reported to show estrogenic activity. Although phytoestrogens have been linked to various beneficial effects in humans (e.g. for reducing the risk of coronary artery disease), phytoestrogens have also been reported to illicit negative reactions. For instance, some phytoestrogens have been reported to aggravate PMS symptoms. As a consequence, the composition of the invention preferably does not exhibit any phytoestrogenic activity, in order to avoid any negative effect which could possibly be linked to the latter.

The composition may take any suitable form known in the art, such as a tablet, a capsule (hard or soft shell), a powder, a liquid such as an ampoule or syrup. In a most preferred embodiment, said composition is a tablet, a capsule, a soft-gel, semi-solid, solid, liquid or a powder.

The active ingredients may be provided in the form of granules, powder or crystalline form, as a component of the compositions according to the invention. This may be achieved by freeze-drying, spray-drying, drum-drying or vacuum drying of the obtained extracts.

In a further preferred embodiment, the compositions according to the invention may comprise excipients chosen from the group of additives such as flowing agents, binding agents, flavors, sweeteners, and/or coating agents.

Solid compositions may also be coated using any pharmaceutically acceptable colored coating agents to improve their appearance and/or facilitate patient identification of the product and unit dosage level.

Said composition comprises vitamin E. In a further embodiment, said composition comprises one or more vitamins selected from the group of vitamin A, vitamin B, vitamin C, vitamin D, vitamin K or any combination thereof. In a preferred embodiment, vitamin E is added to the composition. Vitamin E has been reported to have a beneficial effect on some PMS symptoms and shows anti-oxidative activity.

The composition according to the invention may be a pharmaceutical product, dietary or food supplement, health supplement, medical/dietetic food in an oral form.

In a preferred embodiment, a composition of the invention will comprise:
- between 35 and 60 % by weight of a pollen and pistil extract, preferably originating from a mixture of at least corn (*Zea mays L*.) pollen, rye (*Secale cereale L.*) pollen, cocksfoot (*Dactylis glomerata L*.) pollen, pine *Pinus silvestris L.*) pollen and corn (*Zea mays L*.) pistil; more preferably at least 40% and even more preferably around 50%;
- between 5 and 15% of a saffron extract, more preferably at least 6%;
- between 1 and 5% of vitamin E, more preferably at least 3%; and
- excipients such as bulking, flowing and/or coating agents.

An example of a tablet according to the present invention is the following:

| **Ingredients** | **Quantity mg per tablet (*without coating agent*)** |
|---|---|
| Pollen and pistil extract (corn pollen, rye pollen, cocksfoot pollen, pine pollen and corn pistil) | 320.00 |
| Saffron extract (0.34% Safranal) | 50.0 |
| Vitamin E (anti-oxidant) | 22.2 |
| Microcrystalline cellulose (anticaking aqent) | 232.4 |
| Magnesium Stearate (bulking agent) | 6.0 |
| Silicon dioxide (anticaking agent) | 6.0 |
| **TOTAL (without coating agent)** | **636.6** |

As coating agent, aquapolish is used. The amount of coating agent is at least 2.5% of the bulk tablet weight (approx. 15.9 mg), which makes the total weight of the tablet around 652.5 mg.

The invention is further described by the following non-limiting examples which further illustrate the invention, and are not intended to, nor should they be interpreted to, limit the scope of the invention.

### EXPERIMENTAL DATA AND EXAMPLES

### Example 1:

From the following test results, it appears that compositions on the basis of pollen and pistil extracts only perform poorly when treating or preventing dysphoria and depression in women suffering from PMS.

A clinical trial was performed including 120 patients who suffer from one or more symptoms relating to PMS, which was examined prior to the start of the study. The study was a multicenter out patient randomized, placebo controlled study approved by the Ethical Committee and conducted according to good clinical practice. Each group was subdivided, whereby one group was given a placebo twice a day and a second group a composition on the basis of an extract of pollen and pistil (SERELYS® CLASSIC) originating from corn pollen, rye pollen, cocksfoot pollen, pine pollen and corn pistil, equally twice a day.

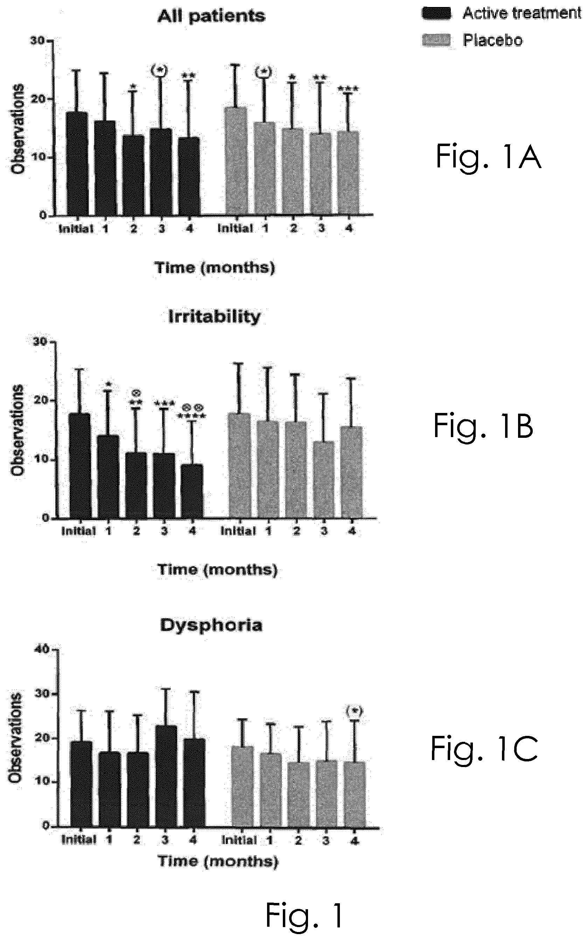

Observer evaluation of all patients enrolled in the trial, without a subdivision for symptoms, showed no significance difference between the group receiving the active treatment and the group receiving the placebo (Figure 1, graph A). This was surprising and contrary to previous studies.

However, it was found that the population of patients could be further subdivided on the basis of their observed symptoms. Whereas one group of patients suffered from classical PMS symptoms such as irritability, anger, short fuse, etc.; a second group reported mainly dysphoria and/or depression as concern.

When looking into results of these subgroups, the group of patients reporting classic symptoms such as irritability showed significant changes between the group receiving the treatment and the placebo. This is especially evident after 2 and 4 months (see figure 1, graph B).

However, within the group reporting mainly dysphoria as most severe symptom, no effect could be observed (see figure 1, graph C, only a borderline alteration within the group was observed, which was not significant when comparing the two groups).

The observer data were further confirmed by the patients' own evaluation, which is summarized below in Table 1 and Table 2.

**Table 1: Personal PMS symptoms score by patients with irritability, short fuse or anger as the most prominent symptom while given a pollen/pistil treatment (n= 29) or placebo (n= 21)**

| | Start of treatment | 1 month | delta value | % | 2 month | delta value | % | 3 month | delta value | % | 4 month | delta value | % |
|---|---|---|---|---|---|---|---|---|---|---|---|---|---|
| **Water retention** | | | | | | | | | | | | | |
| | | | | | | | | | | | | | |
| Active treatment | 3.1(3.1) | 2.0(2.5) | 0.8(2.3) | **28%** | 1.6(2.1) | 1.2(3.8) | **43%** | 1.7(1.9) | 1.1(3.4) | **39%** | 1.9(2.5) | 0.8(3.5)**⁽¹⁾** | **31%** |
| | | | | | | | | | | | | | |
| Placebo | 2.9(3.3) | 2.8(3.2) | -0.0(2.7) | **-0%** | 3.4(3.3) | 0.5(3.7) | **-19%** | 2.8(2.7) | -0.0(2.5) | -1% | 4.2(3.5) | -1.3(3.8)⁽²⁾ | **-44%** |

| **Bloating** | | | | | | | | | | | | | |
|---|---|---|---|---|---|---|---|---|---|---|---|---|---|
| | | | | | | | | | (3) **(4)** | | | ((5)) **(6)** | |
| Active treatment | 4.8(3.3) | 4.0(3.2) | 0.8(3.1) | **16%** | 3.7(3.0) | 1.1(4.1) | **23%** | 2.8(2.5) | 1.9(3.4) | **40%** | 3.3(2.9) | 1.4(3.8) | **29%** |
| Placebo | 4.8(3.1) | 4.4(3.0) | 0.1(2.9) | **2%** | 4.2(3.5) | 0.2(3.2) | **5%** | 4.8(3.4) | -0.4(3.2) | **-8%** | 5.1(3.7) | -0.8(3.8) | **-19%** |

| **Tender breasts** | | | | | | | | | | | | | |
|---|---|---|---|---|---|---|---|---|---|---|---|---|---|
| | | | | | ((7)) | | | | ((8)) | | | (9) | |
| Active treatment | 5.0(3.8) | 4.9(3.8) | 0.0(4.0) | **1%** | 3.5(3.3) | 1.4(4.0) | **28%** | 3.2(3.0) | 1.5(4.0) | **32%** | 3.5(3.2) | 1.2(3.9) | **26%** |
| Placebo | 5.6(3.5) | 5.4(4.0) | 0.2(4.7) | **4%** | 4.0(3.9) | 1.6(4.5) | **29%** | 4.7(3.8) | 0.9(3.2) | **16%** | 5.1(3.9) | 0.3(3.2) | **6%** |

| **Irritability** | | | | | | | | | | | | | |
|---|---|---|---|---|---|---|---|---|---|---|---|---|---|
| | | | (10) **((11))** | | | (12) **(13)** | | | (14) | | | ((15)) **(16)** | |
| Active treatment | 7.0(2.9) | 5.1(3.3) | 1.8(3.7) | **26%** | 4.5(3.4) | 2.3(4.7) | **34%** | 5.1(3.7) | 1.7(3.9) | **25%** | 5.1(3.5) | 1.7(4.1) | **25%** |
| Placebo | 5.9(3.1) | 5.8(3.6) | -0.2(4.4) | **-3%** | 6.3(3.7) | -0.7(3.4) | **-12%** | 4.7(3.1) | 0.9(3.7) | **16%** | 6.5(3.2) | -1.0(3.0) | **-18%** |

| **Depression** | | | | | | | | | | | | | |
|---|---|---|---|---|---|---|---|---|---|---|---|---|---|
| | | | (17) **(18)** | | | (19) **(20)** | | | **((21))** | | | (22) **(23)** | |
| Active treatment | 5.9(3.5) | 4.2(3.7) | 1.6(4.1) | **28%** | 3.5(3.4) | 2.3(4.1) | **40%** | 4.1(3.9) | 1.6(3.9) | **28%** | 3.3(3.3) | 2.4(4.1) | **42%** |
| Placebo | 4.1(3.4) | 4.1(3.5) | -0.3(3.7) | **-7%** | 4.3(3.8) | -0.5(1.5) | **-12%** | 3.1(3.4) | 0.8(3.4) | **20%** | 4.1(3.5) | -0.5(2.7) | **-12%** |

| **Tense** | | | | | | | | | | | | | |
|---|---|---|---|---|---|---|---|---|---|---|---|---|---|
| | | | | | | **((24))** | | | | | | (25) **((26))** | |
| Active treatment | 4.4(3.7) | 3.5(3.5) | 0.5(3.5) | **13%** | 2.3(2.6) | 1.7(4.0) | **42%** | 3.5(3.4) | 0.7(3.3) | **17%** | 2.5(2.7) | 1.7(3.8) | **42%** |
| Placebo | 4.9(3.3) | 4.2(3.7) | 0.6(3.6) | **13%** | 4.6(4.0) | 0.2(2.6) | **4%** | 4.0(3.4) | 0.8(3.1) | **16%** | 4.8(3.9) | -0.2(3.8) | **-5%** |

| **Raw lutheal phase** | | | | | | | | | | | | | |
|---|---|---|---|---|---|---|---|---|---|---|---|---|---|
| | | | (27) | | | (28) **(29)** | | | (30) | | | (31) **(32)** | |
| Active treatment | 5.8(2.7) | 4.3(3.1) | 1.3(3.3) | **23%** | 3.4(2.7) | 2.1 (3.8) | **38%** | 4.2(3.4) | 1.3(3.4) | **24%** | 3.6(2.7) | 1.9(3.5) | **35%** |
| Placebo | 5.0(3.0) | 4.7(3.3) | 0.1(3.4) | **1%** | 5.1 (3.4) | -0.4(1.9) | **-8%** | 3.8(3.0) | 1.0(3.2) | **21%** | 5.2(3.1) | -0.6(2.4) | **-12%** |

| **Headache** | | | | | | | | | | | | | |
|---|---|---|---|---|---|---|---|---|---|---|---|---|---|
| | | | **(33)** | | | (34) **(35)** | | | (36) (37) | | | (38) **(39)** | |
| Active treatment | 4.2(3.7) | 2.6(3.2) | 1.2(4.5) | **31%** | 1.6(2.0) | 2.2(4.1) | **58%** | 1.3(2.4) | 2.6(3.8) | **6 7%** | 2.2(2.6) | 1.8(3.5) | **45%** |
| | | | **(40)** | | | **(41)** | | | | | | **(42)** | |
| Placebo | 2.0(3.0) | 4.2(4.0) | -2.5(3.6) | **-150%** | 3.0(3.9) | -1.3(3.3) | **-80%** | 2.7(3.6) | -1.0(2.5) | **-60%** | 4.6(3.9) | -2.9(3.5) | **-167%** |

| **Libido** | | | | | | | | | | | | | |
|---|---|---|---|---|---|---|---|---|---|---|---|---|---|
| Active treatment | 5.7(2.7) | 5.4(3.2) | 0.1(2.5) | **2%** | 4.6(3.0) | 0.9(3.7) | **17%** | 4.7(3.1) | 0.7(3.5) | **13%** | 4.9(3.5) | 0.6(3.3) | **12%** |
| Placebo | 4.9(3.5) | 5.5(3.5) | -0.2(3.3) | **-3%** | 5.8(3.4) | -0.5(2.8) | **-8%** | 5.3(3.2) | 0.1(2.0) | **1%** | 5.2(3.3) | 0.2(2.4) | **3%** |

| **Intf. with social life** | | | | | | | | | | | | | |
|---|---|---|---|---|---|---|---|---|---|---|---|---|---|
| | | | (43) | | | **(44)** | | | (45) | | | (46) **((42))** | |
| Active treatment | 5.5(3.7) | 3.6(3.2) | 1.6(4.6) | **31%** | 3.0(3.2) | 2.3(4.7) | **43%** | 2.9(3.4) | 2.1(4.4) | **42%** | 2.3(2.9) | 2.9(4.0) | **55%** |
| Placebo | 4.8(3.8) | 3.0(3.0) | 1.4(3.5) | **33%** | 3.7(3.7) | 0.8(3.3) | **17%** | 2.5(2.7) | 1.9(3.5) | **43%** | 3.9(3.4) | 0.7(2.8) | **16%** |

| | | | | | | | | | | | | | |
|---|---|---|---|---|---|---|---|---|---|---|---|---|---|
| 1) p<0.012, 2) p<0.040, 3) p<0.013, 4) p<0.032, 5) p<0.085, 6) p<0.067, 7) p<0.095, 8) p<0.066, 9) p<0.082, 10) p<0.016, 11) p<0.068, 12) p<0.017, 13) p<0.009, 14) p<0.046, 15) p<0.063, 16) p<0.030, 17) p<0.059, 18) p<0.050, 19) p<0.010, 20) p<0.014, 21) p<0.051, 22) p<0.033, 23) p<0.018, 24) p<0.064, 25) p<0.018, 26) p<0.056, 27) p<0.046, 28) p<0.009, 29) p<0.008, 30) p<0.060, 31) p<0.015, 32) p<0.025, 33) p<0.018, 34) p<0.029, 35) p<0.009, 36) p<0.000, 37) p<0.000, 38) p<0.041, 39) p<0.000, 40) p<0.009, 41) p<0.049, 42) p<0.001, 43) p<0.042, 44) p<0.018, 45) p< 0.024, 46) p< 0.004, 47) p<0.079. Bold in table refers to between groups and not bold to within groups differences. Double brackets indicate borderline significance. | | | | | | | | | | | | | |

**Table 2: Personal PMS symptoms score by patients with mood swings, mood reduction and/or enhanced sensitiveness (dysphoria) as the most prominent symptom while given a pollen/pistil treatment (n= 13) or placebo (n= 21)**

| | Start of treatment | 1 month | delta value | % | 2 month | delta value | % | 3 month | delta value | % | 4 month | delta value | % |
|---|---|---|---|---|---|---|---|---|---|---|---|---|---|
| **Water retention** | | | | | | | | | | | | | |
| Active treatment | 4.5(3.8) | 4.5(3.6) | 0.3(3.2) | **5%** | 4.3(3.9) | 0.9(3.8) | **18%** | 5.2(3.1) | 0.6(3.3) | **10%** | 4.8(3.5) | 1.0(3.1) | **18%** |
| Placebo | 3.8(3.4) | 3.8(3.1) | -0.2(2.3) | **-5%** | 3.5(2.9) | 0.1(2.2) | **4%** | 3.0(3.2) | 0.8(2.7) | **22%** | 4.4(3.5) | -0.5(2.1) | **-13%** |

| **Bloating** | | | | | | | | | | | | | |
|---|---|---|---|---|---|---|---|---|---|---|---|---|---|
| Active treatment | 6.1(3.0) | 5.1(3.2) | 1.2(3.6) | **19%** | 4.8(3.6) | 2.1(4.0) | **30%** | 6.1(2.1) | 1.0(3.3) | **14%** | 5.4(3.2) | 1.7(3.3) | **24%** |
| Placebo | 5.7(3.6) | 5.5(3.2) | 0.4(3.3) | **7%** | 5.4(2.7) | 0.5(3.4) | **8%** | 4.6(3.1) | 1.7(3.6)⁽¹⁾ | **27%** | 5.7(3.2) | 0.6(3.8) | **9%** |

| **Tender breasts** | | | | | | | | | | | | | |
|---|---|---|---|---|---|---|---|---|---|---|---|---|---|
| Active treatment | 4.5(3.2) | 4.7(3.6) | -0.1(4.6) | **-2%** | 3.9(3.5) | 0.9(3.9) | **19%** | 4.9(3.6) | 0.5(2.6) | **9%** | 4.6(3.1) | 0.7(3.7) | **14%** |
| Placebo | 3.9(3.9) | 4.0(3.8) | 0.3(2.4) | **7%** | 4.2(3.9) | 0.1(4.2) | **3%** | 3.3(3.6) | 1.2(2.9) | **27%** | 4.2(4.3) | 0.2(3.2) | **5%** |

| **Irritability** | | | | | | | | | | | | | |
|---|---|---|---|---|---|---|---|---|---|---|---|---|---|
| Active treatment | 8.4(2.5) | 6.4(3.5) | 2.1(4.8) | **25%** | 6.5(3.4) | 2.2(4.4) | **25%** | 7.1(2.4) | 1.5(3.7) | **17%** | 6.8(3.3) | 1.8(4.6) | **21%** |
| Placebo | 6.7(3.1) | 6.5(3.2) | -0.0(4.1) | **-0%** | 5.3(3.6) | 1.2(4.4) | **18%** | 6.1(3.2) | 0.5(4.7) | **7%** | 4.9(3.8) | 1.6(4.7) | **25%** |

| **Depression** | | | | | | | | | | | | | |
|---|---|---|---|---|---|---|---|---|---|---|---|---|---|
| Active treatment | 6.1(3.3) | 5.1(4.1) | 1.9(4.9) | **27%** | 4.9(4.2) | 2.7(4.2) | **35%** | 7.3(2.6) | 0.6(3.2) | **8%** | 5.1(3.8) | 2.7(3.8) | **35%** |
| | | | | | | | | | | | | (2) | |
| Placebo | 5.7(3.4) | 5.0(2.8) | 0.5(3.9) | **9%** | 4.5(3.5) | 0.9(4.2) | **17%** | 5.1(3.2) | 0.6(4.8) | **11%** | 3.2(3.4) | 2.5(4.0) | **44%** |

| **Tense** | | | | | | | | | | | | | |
|---|---|---|---|---|---|---|---|---|---|---|---|---|---|
| | (3) | | (4) (5) | | | ((6)) | | | ((7)) (8) | | | | (9) |
| Active treatment | 7.5(2.9) | 4.6(3.6) | 3.3(4.1) | **42%** | 4.6(3.7) | 3.8(4.6) | **45%** | 5.2(3.2) | 3.2(3.5) | **38%** | 4.9(3.1) | 3.5(3.6) | **41%** |
| | | | | | | | | | | | | | |
| Placebo | 5.3(3.1) | 5.8(2.8) | -0.8(3.5) | **-15%** | 3.9(3.5) | 1.2(3.2) | **23%** | 5.2(3.5) | 0.2(3.9) | **3%** | 3.8(3.8) | 1.5(4.0) | 29% |

| **Raw lutheal phase** | | | | | | | | | | | | | |
|---|---|---|---|---|---|---|---|---|---|---|---|---|---|
| | | | | | | ((11)) | | | | | | | ((12)) |
| Active treatment | 7.4(2.4) | 5.4(3.4) | 2.4(4.4) | **31%** | 5.3(3.5) | 2.9(4.3) | **36%** | 6.5(2.2) | 1.8(2.9) | **22%** | 5.6(2.8) | 2.7(3.5) | 33% |
| | | | | | | | | | | | | | ((13)) |
| Placebo | 5.9(2.9) | 5.8(2.8) | -0.1 (3.3) | **-2%** | 4.6(3.3) | 1.1(3.6) | **19%** | 5.5(3.0) | 0.4(4.2) | **7%** | 4.0(3.3) | 1.9(3.8) | 32% |

| **Headache** | | | | | | | | | | | | | ((14)) |
|---|---|---|---|---|---|---|---|---|---|---|---|---|---|
| Active treatment | 5.1(4.2) | 3.6(4.0) | 1.5(5.8) | **30%** | 3.0(3.4) | 2.5(5.2) | **45%** | 3.6(3.6) | 2.5(5.6) | **41%** | 2.8(3.7) | 3.3(5.5) | **54%** |
| Placebo | 4.1(3.7) | 3.1(3.2) | 0.7(2.9) | **19%** | 2.6(3.3) | 1.2(3.7) | **32%** | 4.3(3.7) | -0.5(4.7) | **-13%** | 3.4(3.9) | 0.3(3.3) | **9%** |

| **Libido** | | | | | | | | | | | | | |
|---|---|---|---|---|---|---|---|---|---|---|---|---|---|
| Active treatment | 6.5(3.2) | 5.9(2.9) | 0.5(2.2) | **7%** | 5.7(3.7) | 0.4(2.4) | **7%** | 6.9(3.0) | -0.2(2.5) | **-3%** | 5.7(3.6) | 1.0(3.1) | **15%** |
| Placebo | 6.5(3.4) | 6.4(3.3) | 0.3(2.7) | **5%** | 6.3(3.3) | 0.4(2.9) | **6%** | 5.8(3.2) | 1.3(3.6) | **19%** | 6.0(3.3) | 1.1(3.7) | **16%** |

| **Intf. with social life** | | | | | | | | | | | | | |
|---|---|---|---|---|---|---|---|---|---|---|---|---|---|
| | | | **((15))** | | | | | | ((16)) | | | ((17)) | |
| Active treatment | 5.7(3.9) | 3.0(2.7) | 2.8(4.8) | **48%** | 3.7(3.7) | 2.5(5.2) | **40%** | 3.8(3.8) | 2.2(2.7) | **36%** | 3.7(4.0) | 2.2(3.3) | **37%** |
| | | | ((18)) | | | | | | | | | | |
| Placebo | 4.8(3.2) | 3.9(3.6) | 1.0(2.7) | **21%** | 4.1(3.2) | 0.8(3.8) | **17%** | 4.7(3.8) | 0.5(3.9) | **10%** | 3.7(3.5) | 1.4(3.5) | **28%** |

| | | | | | | | | | | | | | |
|---|---|---|---|---|---|---|---|---|---|---|---|---|---|
| 1) p<0.055, 2) p<0.015, 3) p<0.044, 4) p<0.048, 5) p<0.014, 6) p<0.054, 7) p<0.054, 8) p<0.047, 9) p<0.023, 10) p<0.064, 11) p<0.064, 12) p<0.097, 13) p<0.068, 14) p<0.067, 15) p<0.097, 16) p<0.078, 17) p<0.089, 18) p<0.078. Bold in table refers to between groups and not bold to within groups differences. Double brackets indicate borderline significance. | | | | | | | | | | | | | |

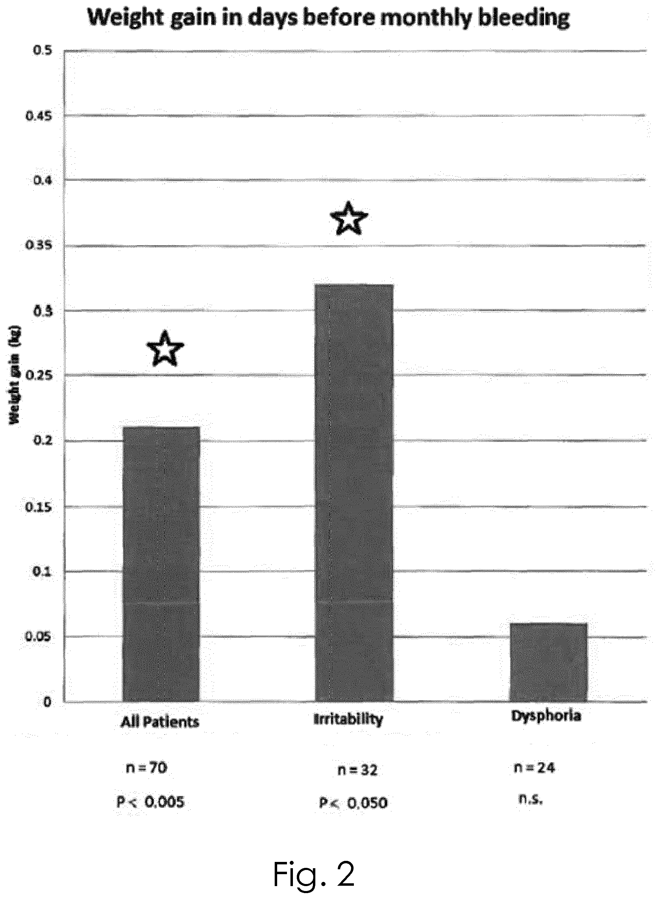

Hence, the results show that whereas a pollen/pistil composition indeed has a significant effect on women suffering from general PMS symptoms, such effect was not seen in the group reporting dysphoria and/or depression as main symptom. It was further found that this group did not report weight gain prior to the start of the menses (see figure 2), which indicates a biochemical/biological reason behind the observed differences.

### Example 2: Use of a composition according to the invention for treatment of Pre Menstrual Syndrome: two patient cases

A 37 years old woman who has not been pregnant for 4 years was suffering from severe PMS, characterized by tender breasts, depressive feelings and mood swings, especially seven days before menstruation. The symptoms were marked as severe. No further health problems were reported or detected. PAP smear was normal, as was the general gynecological examination. Also the blood test results were normal.

In a first instance, classical herbal treatment on the basis of pollen and pistil extract originating from corn pollen, rye pollen, cocksfoot pollen, pine pollen and corn pistil was given for 12 months. Whereas the patient did report an improvement in the tenderness of breasts, no results were observed with regard to the depressive feelings. No other medication was given.

The patient was subsequently given a composition according to the invention as exemplified above (tablet formulation), comprising the pollen and pistil extract originating from corn pollen, rye pollen, cocksfoot pollen, pine pollen and corn pistil, combined with saffron extract. The composition was given to the patient (once a day) for three months.

After three months, the patient was subjected to a questionnaire. From the latter, it showed that the patient observed a reduction in all symptoms of tender breasts, depressive feelings and mood swings, from a severe condition prior to taking the composition towards moderate or mild condition. No side effects were detected. It was decided that the patient should continue the treatment.

### Example 3:

The impact of SERELYS® PMS, a herbal composition based on pollen/pistil extract, saffron and vitamin E, on symptoms from premenstrual tension (PMS) has been tested in a double-blind, randomized, placebo controlled study.

100 hundred women, between 20 and 50 years old with a body mass index below 25 (normal weight), were included in the study. They have suffered PMS (including both irritability and dysphoria) according to criteria designed by Steiner for several years and at least the last 6 months. Their length of cycle was 26 - 30 days. Independent of that, they have been received the required composition for 60 days (two menstrual cycles) after inclusion:
- 50 patients have been treated once-daily with two tablets of a composition comprising a pollen/pistil extract (320 mg per tablet, originating from corn pollen, rye pollen, cocksfoot pollen, and pine pollen and corn pistil) and also comprising saffron and vitamin E;
- 50 patients have been treated once-daily with two tablets of placebo comprising only inert excipients, of similar size, color and smell of the ones above; and
- an additional sub-group of seven patients have received once-daily a composition comprising only 30 mg of saffron and 30 mg of vitamin E.

The patients undergone one consultation initially when the study start, and a second one when finalizing the study after two months of treatment.

Efficacy is analyzed based on the evolution of presence of premenstrual discomforts (particularly on irritability and dysphoria) which are measured by application (initially and after two months of treatment) of two evaluation surveys and a visual analogue scale (VAS):
- Steiner PMTS/S global Self-Rating Scale: it is a survey containing 36 questions that women have to respond with "YES or NO" in connection with the inconveniences of PMS. A score is calculated based on responses given with a maximum of 36.
- Steiner PMTS/Observer-Rating Scale: it is a scale completed by the Doctor in charge of the survey to determine the severity of 10 major disorders of the premenstrual period. Eight disagreements are scored in a scale of 0-4 and two are scored of 0-2. The final score is the sum of scores for the 10 disagreements (maximum = 36).
- Steiner PMTS/VAS: evaluation of premenstrual discomfort. Patients were evaluated on 10 premenstrual disorders (anguish, irritability, dysphoria, headaches, bloating, breast pain, edema, sleep disorders, social and occupational maladjustment) using visual analogue scales from 0 (less severe) to 100 (more severe).
- For satisfaction or overall assessment of women, it will be evaluated according to a scoring system again of 0-4.

Focus is made on irritability and dysphoria symptoms.

### Conclusion:

- Considering the mean values initially and after 2 months treatment, the combination of pollen-pistil extract, saffron and vitamin E should show a statistically significant (p<0.05) improvement by a decrease of at least 30%, advantageously at least around 40%, on the PMTS-S global self-rating scale of PMS.
- More particularly, considering mean values based on PMTS/VAS, the treatment of irritability and dysphoria symptoms should be also statistically significantly (p<0.05) improved by a decrease of at least 30%, for example around respectively 35% and 33% and advantageously respectively of 60.5% and 54.2%, with such a combination.
- The mechanism of action of the combination of the three ingredients (pollen-pistil extract, saffron and vitamin E) of the invention should have an additive impact with a potentiated and synergistic effect on PMS symptoms, and more particularly on dysphoria, with a decrease of at least 30%, advantageously of at least 50%.

Furthermore, when patients are asked for the impact of the treatment as: - No impact; - Minor impact; or - Excellent impact:
- six out of seven patients treated with only saffron and vitamin E answer that it has a minor impact of treatment and one out of seven says excellent impact;
- in the patients treated with pollen and pistil extracts combined with both saffron and vitamin E, all patients answer that it has an excellent impact.

## Claims

1. A composition for use in treating dysphoria, depression and/or mood swings relating to premenstrual syndrome (PMS) wherein said composition comprises as first active ingredient:
- a pollen and pistil extract derived from plants belonging to the *Graminae* and/or *Pinacea* family, preferably from plant material originating from corn (*Zea mays L*)*.,* rye (*Secale cereale L*.), cocksfoot (*Dactylis glomerata L*.), pine (*Pinus sylvestris L*.) or a mixture thereof,
- at least one second active ingredient, being a source of safranal and/or (picro)crocin wherein said source of safranal and/or (picro)crocin is saffron or a derivative of saffron, and
- vitamin E.

2. The composition for use according to claim 1, wherein said pollen and pistil extract is derived from a mixture of at least two separate extracts, whereby a first extract originates from corn pistil and from pollen selected from corn, rye, cocksfoot, pine or any combination thereof; and a second extract originating from corn pollen and pistil.

3. The composition for use according to claim 2, wherein said first extract originates from a mixture of at least corn pollen, rye pollen, cocksfoot pollen, pine pollen and corn pistil.

4. The composition for use according to claim 1, wherein said pollen and pistil extract comprises a first and a second extract, whereby said fist extract is originating from pollen selected from corn, rye, pine or a combination thereof and a second extract, whereby said second extract is originating from corn pistil and pollen selected from corn, rye, cocksfoot, pine or any combination thereof.

5. The composition for use according to claim 1, wherein said derivative of saffron is a saffron extract.

6. The composition for use according to claim 5, wherein saffron extract is derived from *Crocus sativus.*

7. The composition for use according to any of the previous claims, wherein said composition is a pharmaceutical product, dietary or food supplement, health supplement, medical/dietic food in an oral form.

8. The composition for use according to any of the previous claims, wherein said composition is a tablet, a capsule, soft gel, semi-solid, solid, liquid or a powder.

9. The composition for use according to any of the previous claims, wherein said composition further comprises one or more vitamins selected from the group of vitamin A, vitamin B, vitamin C, vitamin D, vitamin K or any combination thereof.

10. The composition for use according to any of the previous claims, comprising excipients chosen from the group of flowing agents, binding agents, flavors, sweeteners and/or coating agents.

## Patentansprüche

1. Zusammensetzung zur Verwendung bei der Behandlung von Dysphorie, Depression und/oder Stimmungsschwankungen im Zusammenhang mit prämenstruellem Syndrom (PMS), wobei die Zusammensetzung als ersten Wirkstoff umfasst:
- einen Pollen- und Stempel-Extrakt, der aus Pflanzen gewonnen wird, die zu der Graminae und/oder der Pinacea Familie gehören, vorzugsweise aus Pflanzenmaterial, das von Mais (*Zea mays L*.), Roggen (*Secale cereale L*.), Knäuelgras (*Dactylis glomerata L*.), Waldkiefer (*Pinus sylvestris L*.) oder einer Mischung davon stammt,
- mindestens einen zweiten Wirkstoff, der eine Quelle für Safranal und/oder (Pico)crocin ist, wobei die Quelle für Safranal und/oder (Pico)crocin Safran oder ein Safranderivat ist, und
- Vitamin E.

2. Zusammensetzung zur Verwendung nach Anspruch 1, wobei der Pollen- und Stempel-Extrakt aus einer Mischung von mindestens zwei separaten Extrakten abgeleitet ist, wobei ein erster Extrakt von einem Maisstempel und von Pollen stammt, der aus Mais, Roggen, Knäuelgras, Kiefer oder irgendeiner Kombination davon ausgewählt ist; und ein zweiter Extrakt von Maispollen und -stempel stammt.

3. Kombination zur Verwendung nach Anspruch 2, wobei der erste Extrakt aus einer Mischung von zumindest Maispollen, Roggenpollen, Knäuelgraspollen, Kiefernpollen und Maisstempel stammt.

4. Zusammensetzung zur Verwendung nach Anspruch 1, wobei der Pollen- und Stempel-Extrakt einen ersten und einen zweiten Extrakt umfasst, wobei der erste Extrakt von Pollen stammt, der ausgewählt ist aus Mais, Kiefer oder einer Kombination davon, und einen zweiten Extrakt, wobei der zweite Extrakt von Maisstempel und von Pollen stammt, der ausgewählt ist aus Mais, Roggen, Knäuelgras, Kiefer oder irgendeiner Kombination davon.

5. Zusammensetzung zur Verwendung nach Anspruch 1, wobei das Safranderivat Safranextrakt ist.

6. Zusammensetzung zur Verwendung nach Anspruch 5, wobei Safranextrakt von *Crocus sativus* abgeleitet ist.

7. Zusammensetzung zur Verwendung nach einem der vorangehenden Ansprüche, wobei die Zusammensetzung ein pharmazeutisches Produkt, ein Diät- oder Nahrungsergänzungsmittel, ein Gesundheitszusatz, ein medizinisches/diätisches Nahrungsmittel in oraler Form ist.

8. Zusammensetzung zur Verwendung nach einem der vorangehenden Ansprüche, wobei die Zusammensetzung eine Tablette, eine Kapsel, ein weiches Gel, halbfest, fest, flüssig oder pulverisiert ist.

9. Zusammensetzung zur Verwendung nach einem der vorangehenden Ansprüche, wobei die Zusammensetzung ferner ein oder mehrere Vitamine umfasst, die ausgewählt sind aus der Gruppe von Vitamin A, Vitamin B, Vitamin C, Vitamin D, Vitamin K oder irgendeiner Kombination davon.

10. Zusammensetzung zur Verwendung nach einem der vorangehenden Ansprüche, Hilfsstoffe umfassend, die ausgewählt sind aus der Gruppe der Fließmittel, Bindemittel, Geschmacksmittel, Süßungsmittel und/oder Beschichtungsmittel.

## Revendications

1. Composition pour son utilisation dans le traitement de la dysphorie, de la dépression et/ou des troubles de l'humeur liés au syndrome prémenstruel (SPM), dans laquelle ladite composition comprend en tant que premier ingrédient actif :
- un extrait de pollen et de pistil dérivé de plantes appartenant à la famille des *Graminae* et/ou des *Pinacea,* de préférence d'un matériel végétal provenant de maïs (*Zea mays L*.), de seigle (*Secale cereale L*.), de dactyle (*Dactylis glomerata* L.), de pin (*Pinus sylvestris* L.) ou d'un mélange de ceux-ci,
- au moins un deuxième ingrédient actif, qui est une source de safranal et/ou de (picro)crocine, dans laquelle ladite source de safranal et/ou de (picro)crocine est du safran ou un dérivé du safran, et
- de la vitamine E.

2. Composition pour son utilisation selon la revendication 1, dans laquelle ledit extrait de pollen et de pistil est dérivé d'un mélange d'au moins deux extraits séparés, un premier extrait provenant du pistil de maïs et d'un pollen choisi parmi le maïs, le seigle, le dactyle, le pin ou toute combinaison de ceux-ci ; et un second extrait provenant de pollen et de pistil de maïs.

3. Composition pour son utilisation selon la revendication 2, dans laquelle ledit premier extrait provient d'un mélange d'au moins de pollen de maïs, de pollen de seigle, de pollen de dactyle, de pollen de pin et de pistil de mais.

4. Composition pour son utilisation selon la revendication 1, dans laquelle ledit extrait de pollen et de pistil comprend un premier et un second extrait, ledit premier extrait provenant de pollen choisi parmi le maïs, le seigle, le pin ou une combinaison de ceux-ci, et un second extrait, ledit second extrait provenant du pistil de maïs et d'un pollen choisi parmi le maïs, le seigle, le dactyle, le pin ou une combinaison de ceux-ci.

5. Composition pour son utilisation selon la revendication 1, dans laquelle ledit dérivé de safran est un extrait de safran.

6. Composition pour son utilisation selon la revendication 5, dans laquelle l'extrait de safran est dérivé de *Crocus sativus.*

7. Composition pour son utilisation selon l'une des revendications précédentes, dans laquelle ladite composition est un produit pharmaceutique, un complément alimentaire ou diététique, un supplément de santé, un aliment médical/diététique sous forme orale.

8. Composition pour son utilisation selon l'une des revendications précédentes, dans laquelle ladite composition est un comprimé, une gélule, une capsule molle, un semi-solide, un solide, un liquide ou une poudre.

9. Composition pour son utilisation selon l'une des revendications précédentes, dans laquelle ladite composition comprend en outre une ou plusieurs vitamines sélectionnées parmi le groupe de vitamine A, vitamine B, vitamine C, vitamine D, vitamine K, ou toute combinaison de celles-ci.

10. Composition pour son utilisation selon l'une des revendications précédentes, comprenant des excipients sélectionnés parmi le groupe des agents d'écoulement, agents de liaison, arômes, édulcorants et/ou agents d'enrobage.
